# EUROPEAN PATENT APPLICATION

(11) **EP 3 670 487 A1**
(43) Date of publication of application: **24.06.2020**
(21) Application number: 18213700.0
(22) Date of filing: 18.12.2018
(51) Int. Cl.: C07C 45/64, C07D 249/08, C07C 49/84

(54) **PROCESS FOR THE PREPARATION OF SUBSTITUTED PHENOXYPHENYL KETONES**

(71) Applicant: BASF Agro B.V., 6835 EA Arnhem (NL)
(72) Inventor: The designation of the inventor has not yet been filed
(74) Representative: BASF IP Association

(57) **Abstract**

The present invention relates to a process for providing substituted phenoxyphenyl ketones. The invention is further relates to a process for the preparation of substituted 1-[4-phenoxy-2-(halogenalkyl)phenyl]-2-(1,2,4-triazol-1-yl)ethanol compounds with fungicidal activity.

## Description

The present invention relates to a process for providing substituted phenoxyphenyl ketones. The invention is further relates to a process for the preparation of substituted 1-[4-phenoxy-2-(halogenalkyl)phenyl]-2-(1,2,4-triazol-1-yl)ethanol compounds with fungicidal activity.

WO 2013/007767 describes the above mentioned triazole compounds, their fungicidal activity and their synthesis via substituted phenoxyphenyl ketones. These compounds are highly efficient fungicides. Therefore, there is an ongoing need for processes that easily make them available.

WO 2014/108286, WO 2015/091045, WO2016/005211 and WO 2016/202807 describe process steps and processes for the synthesis of substituted 1-[4-phenoxy-2-(halogenalkyl)phenyl]-2-(1,2,4-triazol-1-yl)ethanol compounds.

The present invention provides a process for the preparation of substituted phenoxyphenyl ketones and fungicidal 1-[4-phenoxy-2-(halogenalkyl)phenyl]-2-(1,2,4-triazol-1-yl)ethanol derivatives utilizing a different starting material.

It was an object of the present invention to provide an industrially simple process for the preparation of substituted phenoxyphenyl ketones in good yields. In addition, the process should be environmentally friendly in order to reduce unfavorable environmental effects. A further object of the present invention was to provide an improved process for the synthesis of 1-[4-phenoxy-2-(halogenalkyl)phenyl]-2-(1,2,4-triazol-1-yl)ethanol compounds.

It has now surprisingly been found a highly efficient process for the synthesis of substituted phenoxyphenyl ketones, and thus, an efficient synthesis of triazole compounds with fungicidal activity.

The present invention thus relates to a process for the preparation of the compound of formula (V) 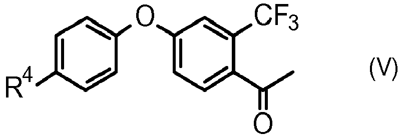 wherein
- R⁴: is halogen;
comprising the following step:
(iii) reacting a compound of formula (III) 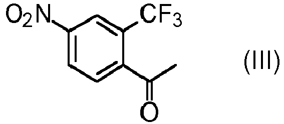 with a phenol derivative of formula (IV) 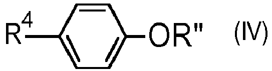 wherein
   - R": is hydrogen or an alkali metal ion;
   - R⁴: is as defined for compounds of formula (V);
   in the presence of a base.

The present invention further relates to the process for the preparation of the substituted phenoxyphenyl ketones (V): 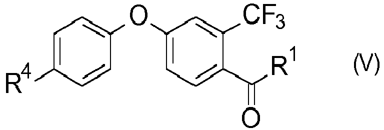 wherein
- R¹: is C₁-C₆-alkyl or C₃-C₈-cycloalkyl;
- R⁴: is halogen
comprising the following steps:
(iii) as described herein 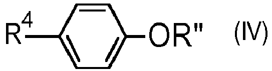 wherein the variables have the following meanings:
   - R": is hydrogen or an alkali metal ion;
   - R⁴: is as described for compounds of formula (V);
   to give the compounds of formula (V).

The present invention further relates to a process for the preparation of triazole compounds of the formula (T): 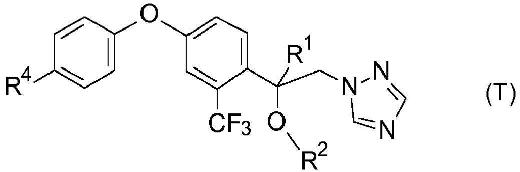 wherein R¹ and R⁴ are as described for the compounds of formula (V),
- R²: is hydrogen, C₁-C₆-alkyl, C₂-C₆-alkenyl, C₂-C₆-alkynyl, C₃-C₈-cycloalkyl, C₃-C₈-cycloalkyl-C₁-C₆-alkyl, phenyl, phenyl-C₁-C₄-alkyl, phenyl-C₂-C₄-alkenyl or phenyl-C₂-C₄-alkynyl;
wherein the aliphatic moieties of R² are not further substituted or do carry one, two, three or up to the maximum possible number of identical or different groups R^{2a} which independently are selected from:
- R^{2a}: halogen, OH, CN, nitro, C₁-C₄-alkoxy, C₃-C₈-cycloalkyl, C₃-C₈-halocycloalkyl and C₁-C₄-halogenalkoxy;
wherein the cycloalkyl and/or phenyl moieties of R² are not further substituted or do carry one, two, three, four, five or up to the maximum number of identical or different groups R^{2b} which independently are selected from:
R^{2b} halogen, OH, CN, nitro, C₁-C₄-alkyl, C₁-C₄-alkoxy, C₁-C₄-halogenalkyl, C₃-C₈-cycloalkyl, C₃-C₈-halocycloalkyl and C₁-C₄-halogenalkoxy; comprising the following steps:
(iii) as described herein;
(iv) converting the ketone of the formula (V) to oxiranes (O); 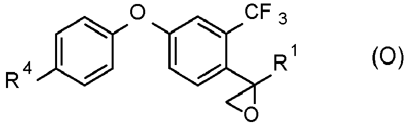
(v) reacting the oxirane (O) with 1H-1,2,4-triazole in the presence of a base to obtain compounds (T), wherein R² is hydrogen (compounds T-1);
   and, for obtaining compounds wherein R² is different from hydrogen:
(vi) derivatizing the compound of formula (T-1) of step (v) under basic conditions with R²-LG, wherein LG is a nucleophilically replaceable leaving group; to result in compounds (T-2).

Further embodiments of the invention are evident from the claims, the description and the examples. It is to be understood that the single features of the subject matter of the invention described herein can be applied not only in the combination given in each particular case but also in other combinations, without leaving the scope of the invention.

In the definitions of the variables given herein, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Likewise, the term "C₂-C₄-alkyl" refers to a straight-chained or branched alkyl group having 2 to 4 carbon atoms, such as ethyl, propyl (n-propyl), 1-methylethyl (iso-propoyl), butyl, 1-methylpropyl (sec.-butyl), 2-methylpropyl (iso-butyl), 1,1-dimethylethyl (tert-butyl).

The term "C₁-C₆-haloalkyl" or "C₁-C₆-halogenalkyl" refers to an alkyl group having 1 or 6 carbon atoms as defined above, wherein some or all of the hydrogen atoms in these groups may be replaced by halogen atoms as mentioned above. A preferred embodiment of a C₁-C₆-haloalkyl is a C₁-C₂-haloalkyl. Representative C₁-C₂-haloalkyl groups include chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl.

The term "C₂-C₆-alkenyl" refers to a straight-chain or branched unsaturated hydrocarbon radical having 2 to 6 carbon atoms and a double bond in any position, e.g. ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl. Likewise, the term "C₂-C₆-alkenyl" refers to a straight-chain or branched unsaturated hydrocarbon radical having 2 to 6 carbon atoms and a double bond in any position.

The term "C₂-C₆-alkynyl" refers to a straight-chain or branched unsaturated hydrocarbon radical having 2 to 6 carbon atoms and containing at least one triple bond, such as ethynyl, prop-1-ynyl, prop-2-ynyl (propargyl), but-1-ynyl, but-2-ynyl, but-3-ynyl, 1-methyl-prop-2-ynyl. Likewise, the term "C₂-C₆-alkynyl" refers to a straight-chain or branched unsaturated hydrocarbon radical having 2 to 6 carbon atoms and at least one triple bond.

The term "C₃-C₈-cycloalkyl" refers to monocyclic saturated hydrocarbon radicals having 3 to 8 carbon ring members, such as cyclopropyl, cyclobutyl, cyclopentyl, cyclohexyl, cycloheptyl or cyclooctyl.

The term "C₃-C₈-halocycloalkyl" or "C₃-C₈-halogencycloalkyl" refers to monocyclic saturated hydrocarbon radicals having 3 to 8 carbon ring members as defined above, wherein some or all of the hydrogen atoms in these groups may be replaced by halogen atoms as mentioned above.

The term "C₃-C₆-cycloalkyl-C₁-C₆-alkyl" refers to alkyl having 1 to 6 carbon atoms (as defined above), wherein one hydrogen atom of the alkyl radical is replaced by a cycloalkyl radical having 3 to 6 carbon atoms (as defined above).

The term "C₁-C₆-alkoxy" refers to a straight-chain or branched alkyl group having 1 to 6 carbon atoms which is bonded via an oxygen at any position in the alkyl group. Examples are "C₁-C₄-alkoxy" groups, such as methoxy, ethoxy, n-propoxy, 1-methylethoxy, butoxy, 1-methyhpropoxy, 2-methylpropoxy or 1,1-dimethylethoxy.

The term "C₁-C₆-haloalkoxy" or "C₁-C₆-halogenalkoxy" refers to a C₁-C₆-alkoxy radical as defined above, wherein some or all of the hydrogen atoms in these groups may be replaced by halogen atoms as mentioned above. A preferred embodiment of a C₁-C₆-haloalkoxy is a C₁-C₄-haloalkoxy. Examples of C₁-C₄-haloalkoxy groups include substituents, such as OCH₂F, OCHF₂, OCF₃, OCH₂Cl, OCHCl₂, OCCl₃, chlorofluoromethoxy, dichlorofluoromethoxy, chlorodifluoro-methoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloro¬ethoxy, OC₂F₅, OCF₂CHF₂, OCHF-CF₃, 2-fluoropropoxy, 3-fluoropropoxy, 2,2-difluoropropoxy, 2,3-difluoro¬propoxy, 2 chloropropoxy, 3-chloropropoxy, 2,3-dichloropropoxy, 2-bromo¬propoxy, 3 bromopropoxy, 3,3,3-trifluoropropoxy, 3,3,3-trichloropropoxy, OCH₂-C₂F5, OCF₂-C₂F5, 1-fluoromethyl-2-fluoroethoxy, 1-chloromethyl-2-chloroethoxy, 1-bromomethyl-2-bromo¬ethoxy, 4-fluorobutoxy, 4-chlorobutoxy, 4-bromobutoxy or nonafluorobutoxy.

The term "phenyl-C₁-C₄-alkyl" refers to alkyl having 1 to 6 carbon atoms (as defined above), wherein one hydrogen atom of the alkyl radical is replaced by a phenyl radical. Likewise, the terms "phenyl-C₂-C₄-alkenyl" and "phenyl-C₂-C₄-alkynyl" refer to alkenyl and alkynyl, respectively, wherein one hydrogen atom of the aforementioned radicals is replaced by a phenyl radical.

According to step (iii) of the process, compounds (III) are reacted with a phenol of formula (IV) 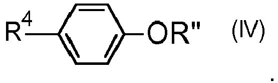
R" in formula (IV) is hydrogen or an alkali metal cation, preferably Na or K.
R⁴ in formula (IV) is halogen, preferably F or Cl, in particular Cl.

Compound (III) can be prepared as known to the skilled person or as described below.

Compounds (IV) can be synthesized as known to the skilled person or are also commercially available.

If R" in formula (IV) is hydrogen, the reaction is carried out in the presence of a base. The base can be organic or inorganic base.

According to one embodiment, the base used in step (iii) is an inorganic base. Examples of suitable inorganic bases are hydroxides, carbonates, hydrocarbonates, phosphates and hydrophosphates of alkali or earth alkali metals or any mixtures thereof. Examples of the suitable inorganic bases are NaOH, KOH, CsOH, Na₂CO₃, K₂CO₃, Cs₂CO₃, NaHCO₃, KHCO₃, Na₃PO₄, K₃PO₄, Na₂HPO₄, K₂HPO₄, NaH₂PO₄, KH₂PO₄.

The inorganic base is preferably selected from NaOH, KOH, CsOH, Na₂CO₃, K₂CO₃ and Cs₂CO₃, more preferably from Na₂CO₃, K₂CO₃ and Cs₂CO₃. According to one specific embodiment, the inorganic base is Na₂CO₃. According to another specific embodiment, the inorganic base is K₂CO₃. According to another specific embodiment, the inorganic base is Cs₂CO₃.

The hydroxides and carbonates can be used alone or in the presence of 0.05-0.2 equivalents of CsF or CsCI per 1 equivalent of the hydroxide or carbonate base.

According to another embodiment, the base used in step (iii) is an organic base. Examples of suitable organic bases are alkoxides, acetates, tertiary amines, pyridine, substituted pyridines, or any mixture thereof.

Examples of suitable alkoxides are NaOCH₃, KOC(CH₃)₃ and the like.

According to another specific embodiment the organic base is selected from acetates. Examples of suitable acetates are CH₃COONa, CH₃COOK, CH₃COOCs.

Examples of suitable tertiary amines are tri-(C₁-C₆)-alkylamines such as trimethylamine, triethylamine, tributylamine and N,N-diisopropylethylamine; di-(C₁-C₆)-alkyl-phenylamines such as N,N-dimethylaniline and N,N-diethylaniline; N-methyl imidazole, N,N-dimethylaminopyridine and the like.

Examples of suitable substituted pyridines are collidine, lutidines, picolines, N,N-dimethylaminopyridine and the like.

The base can be used in solid form or as a solution, e.g. as aqueous solution.

The molar ratio of the base to compound (III) is generally from 0.5:1 to 4:1, preferably from 0.5:1 to 3.5:1, more preferably from 1:1 to 3:1, most preferably from 1:1 to 2:1.

The process step (iii) according to the present invention can be carried out in the presence of a catalyst. The catalyst is preferably a Cu(I), Cu(II) or Fe(III) catalyst. Examples of suitable catalysts are Cu(I)Cl, Cu(II)Cl₂, CuSO₄•5H₂0, Cu(OAc)₂ and FeCl₃. According to one specific embodiment the catalyst is Cu(OAc)₂.

The molar ratio of the catalyst to compound (III) is generally from 0.005:1 to 0.5:1, preferably from 0.075:1 to 0.4:1, more preferably from 0.01:1 to 0.3:1, most preferably from 0.05:1 to 0.1:1.

Examples for appropriate solvents for step (iii) of the inventive process are aprotic organic solvents such as for example dimethyl formamide (DMF), N-methyl pyrrolidone (NMP), dimethyl imidazolidinone (DMI), toluene, o-xylene, m-xylene, p-xylene; ethers such as monoglyme or diglyme; dimethylactamide (DMA), DMSO and any mixtures thereof. In particular DMF, NMP, toluene and DMA or any mixtures, more specifically DMF, are particularly suitable.

According to one specific embodiment, the solvent used in step (iii) contains not more than 3 eq DMF in relation to 1 eq of the phenol of formula (IV), preferably not more than 2.8 eq to 1 eq of the phenol of formula (IV), more preferably not more than 2.6 eq to 1 eq of the phenol of formula (IV), most preferably not more than 2.4 eq to 1 eq of the phenol of formula (IV), specifically not more than 2.2 eq DMF to 1 eq of the phenol of formula (IV).

The reagents for step (iii) are preferably added at ambient temperature and the reaction temperature is then elevated, wherein the reaction temperature after the reagents have been added is preferably held at a maximum of 150°C, in particular at a maximum of 140°C, more preferably at a maximum of 130°C. Generally, it is preferred to have a reaction temperature of 20°C to 135°C, in particular 50°C to 135°C, more particularly 100°C to 130°C.

After step (iii), a work-up of the reaction mixture can be carried out by procedures known in a general manner to the person skilled in the art. Generally, water is added and the aqueous phase is extracted with a suitable solvent, e.g. toluene or o-xylene. The raw product obtained after evaporation of the solvent(s) can directly be used in a further step, if desired. However, the raw product can also be further worked up and/or purified as generally known to the skilled person.

According to one embodiment of the invention, after completion of the reaction, most of the solvent (e.g. DMF or toluene) is removed from the reaction mixture, preferably under reduced pressure. Then, a suitable organic solvent, such as, for example, toluene or o-xylene, is added together with water. According to the inventive process, it may be favorable to carry out one to three, preferably two extractions of the aqueous phase.

By means of the inventive process, the compounds of formula (V) can be prepared in surprisingly high yields. The yields of step (iii) are preferably at least 60%, more preferably at least 70 %, even more preferred at least 80%, most preferred at least 90%.

The compounds of formula (III) can be prepared by a process comprising the following steps:
(i) reacting a compound of formula (II) 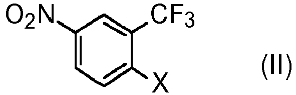 wherein X is halogen;
   with nitroethane in the presence of a base to give the compound of formula (I) 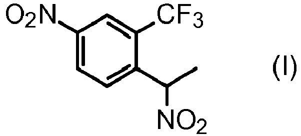
(ii) converting a compound of formula (I) into the compound of formula (III) in the presence of an oxidant.

The compounds of formula (II) can be synthesized as known to the skilled person or are also commercially available.

According to the present invention, the variable X of the compound of formula (II) used in step (i) is halogen. X is preferably selected from Br or Cl, more preferably X is Cl. According to one specific embodiment, X is Br. According to another specific embodiment, X is Cl.

In the process step (i) according to the present invention, the compound (II) is reacted with nitroethane.

The molar ratio of the nitroethane to compound (II) is generally in the range from 10:1 to 1:10, preferably from 3:1 to 1:3, more preferably from 2:1 to 1:2, most preferably from 1.5:1 to 1:1.5. The molar ratio of the nitroethane to compound (II) can also be in the range from 1.3:1 to 1:1.3.

The step (i) according to the process of the present invention is carried out in the presence of a base. The base used in step (i) can be an inorganic base or an organic base.

According to one embodiment the base is an inorganic base. Suitable inorganic bases are hydroxides, carbonates, hydrocarbonates, phosphates and hydrophosphates of alkali or earth alkali metals or any mixtures thereof. Examples of the suitable inorganic bases are NaOH, KOH, CsOH, Na₂CO₃, K₂CO₃, Cs₂CO₃, NaHCO₃, KHCO₃, CsHCO₃, Na₃PO₄, K₃PO₄, Na₂HPO₄, K₂HPO₄, NaH₂PO₄ or KH₂PO₄.

According to one embodiment, the bases are selected from hydroxides such as NaOH, KOH, or CsOH. According to one specific embodiment, the inorganic base is NaOH. According to another specific embodiment, the inorganic base is KOH. According to another specific embodiment, the inorganic base is CsOH. Hydroxides can be used alone or in the presence of 0.05-0.2 equivalents of CsF or CsCI per 1 equivalent of the hydroxide base.

According to another embodiment, the bases are selected from carbonates, such as Na₂CO₃, K₂CO₃, or Cs₂CO₃. According to one specific embodiment, the inorganic base is Na₂CO₃. According to another specific embodiment, the inorganic base is K₂CO₃. According to another specific embodiment, the inorganic base is Cs₂CO₃. Carbonates can be used alone or in the presence of 0.05-0.2 equivalents of CsF or CsCI per 1 equivalent of the carbonate.

According to another embodiment, the bases are selected from hydrocatbonates such as Na-HCOO₃, KHCO₃, CsHCO₃. According to another specific embodiment, the inorganic base is Na-HCO₃. According to another specific embodiment, the inorganic base is KHCO₃. According to another specific embodiment, the inorganic base is CsHCO₃. Hydrocarbonates each can be used alone or in the presence of 0.05-0.2 equivalents of CsF or CsCI per 1 equivalent of the hydrocarbonate.

According to another embodiment, the bases are selected from phosphates, such as Na₃PO₄ or K₃PO₄; or hydrophosphates such as Na₂HPO₄, K₂HPO₄, NaH₂PO₄, KH₂PO₄. According to one specific embodiment, the inorganic base is Na₃PO₄. According to another specific embodiment, the inorganic base is K₃PO₄. According to another specific embodiment, the inorganic base is Na₂HPO₄. According to another specific embodiment, the inorganic base is K₂HPO₄. According to another specific embodiment, the inorganic base is NaH₂PO₄. According to another specific embodiment, the inorganic base is KH₂PO₄.

The base can be used in a solid form, e.g. solid pellets, flakes, micropills or powder, or as a solution, e.g. as aqueous solution.

According to another embodiment the base is an organic base. Examples of suitable organic bases are alkoxides, acetates, tertiary amines, quaternary ammonium salts, amidines, guanidine derivatives, pyridine, substituted pyridines, bicyclic amines or any mixture thereof.

According to one specific embodiment the organic base is selected from alkoxides. Examples of suitable alkoxides are (C₁-C₁₀)-alkyl-alkoxydes, such as NaOCH₃, KOCH₃, NaOC₂H₅, KOC₂H₅, NaOC₃H₇, KOC₃H₇, NaOCH(CH₃)₂, KOCH(CH₃)₂, NaOC(CH₃)₃, KOC(CH₃)₃, sodium *tert-*amylate, potassium *tert*-amylate, sodium 3,7-dimethyl-3-octylate, potassium 3,7-dimethyl-3-octylate and the like. Preferable alkoxide bases are selected from C₁-C₄-alkyl-alkoxydes, such as NaOCH₃, KOCH₃, NaOC₂H₅, KOC₂H₅, NaOC₃H₇, KOC₃H₇, NaOCH(CH₃)₂, KOCH(CH₃)₂, NaOC(CH₃)₃a, KOC(CH₃)₃. Most preferable alkoxides are NaOCH₃ and KOC(CH₃)₃.

According to another specific embodiment the organic base is selected from acetates. Examples of suitable acetates are CH₃COONa, CH₃COOK, CH₃COOCs.

According to another specific embodiment the organic base is selected from tertiary amines. Examples of suitable tertiary amines are tri-(C₁-C₆)-alkylamines such as trimethylamine, triethylamine, tributylamine and N,N-diisopropylethylamine; di-(C₁-C₆)-alkyl-phenylamines such as N,N-dimethylaniline and N,N-diethylaniline; N-methyl imidazole, N,N-dimethylaminopyridine and the like. Preferable tertiary amine bases are selected from trimethylamine, triethylamine, tributylamine and N,N-diisopropylethylamine.

According to another specific embodiment the organic base is selected from quaternary ammonium salts. Example of a suitable quaternary ammonium salt is tetrabutylammonium fluoride (TBAF).

According to another specific embodiment the organic base is selected from amidines. Examples of suitable amidines are 1,8-diazabicyclo[5.4.0]undec-7-en (DBU) and 1,5-diazabicyclo-[4.3.0]non-5-ene (DBN).

According to another specific embodiment the organic base is selected from guanidine derivatives. Examples of suitable guanidine derivatives tetramethyl guanidine (TMG), 1,5,7-triazabicyclo[4.4.0]dec-5-ene (TBD) and the like.

According to another specific embodiment the organic base is selected from pyridine and substituted pyridines. Pyridine is preferred. Examples of suitable substituted pyridines are collidine, lutidines, picolines, N,N-dimethylaminopyridine and the like.

According to another specific embodiment the organic base is selected from bicyclic amines. Example of suitable bicyclic amines is 1,4-diazabicyclo[2.2.2]octane (DABCO) and the like.

According to one embodiment, the organic base is selected from alkoxides or tertiary amines.

According to another embodiment, the organic base is selected from guanidine derivatives or bicyclic amines.

The molar ratio of the base to compound (II) is generally in the range from 10:1 to 0.8:1, preferably from 5:1 to 1:1, more preferably from 4:1 to 1:1, most preferably from 3:1 to 1:1.

The step (i) according to the present invention can be carried out with or without a solvent.

According one embodiment, the reaction is carried out without a solvent.

According another embodiment, the reaction is carried out in an inert organic solvent. Examples of suitable inert organic solvents are aromatic hydrocarbons, such as benzene, toluene, ethylbenzene, cymene, mesitylene, o-xylene, m-xylene and p-xylene; N-nethyl-2-pyrrolidon (NMP), dimethylformamide (DMF), tetrahydrofurane (THF), alcohols, such as ethanol, 1-propanol, 2-propanol, 1-butanol and 2-butanol; ethers, such as 1-4 dioxane, dimethylglycol (monoglyme) and bis(2-methoxyethyl) (diglyme); esters such as alkyl acetates e.g. ethyl acetate, butyl acetate, iso-propyl acetate; dimethylsulfoxid (DMSO), dimethylacetamid (DMAC) or any mixture thereof. Preferably, the solvent is selected from toluene, ethyl acetate, butyl acetate, iso-propyl acetate, NMP, DMF, DMSO, DMAC or any mixtures thereof. According to one specific embodiment, the solvent is toluene. According to another specific embodiment, the solvent is ethyl acetate. According to another specific embodiment, the solvent is butyl acetate. According to another specific embodiment, the solvent is iso-propyl acetate. According to another specific embodiment, the solvent is NMP. According to another specific embodiment, the solvent is DMF. According to another specific embodiment, the solvent is DMSO. According to another specific embodiment, the solvent is DMAC.

The reagents in step (i) can be added at ambient temperature or under cooling. According to one embodiment the reagents are added at ambient temperature. According to another embodiment the reagents are added at a temperature between 10 and 20°C. After the reagents have been added the reaction temperature is preferably held at ambient temperature. The temperature can be also elevated to 30-50°C.

The order of adding the reagents to the reaction mixture is variable.

According to one embodiment, compound (II) and nitroethane are added to the base. Compound (II) and nitroethane can be added separately or in form of a mixture thereof. According to one specific embodiment, compound (II) and nitroethane are added to the base separately. They can be added subsequently or simultaneously. According to another embodiment, compound (II) and nitroethane are mixed together and then added to the base. The addition of compound (II) and nitroethane, as single compounds or in form of the mixture thereof, can be done in one portion or gradually, e.g. dropwise or in several portions. According to one embodiment, the addition is made in one portion. According to another embodiment the addition is made gradually, e.g. dropwise or in several portions. The base is preferably suspended in sufficient solvent and is stirred during the addition of the reagents.

According to a further embodiment, the base is added to the mixture of compound (II) and nitroethane. The base can be added in one portion or gradually, e.g. dropwise or in several portions. According to one embodiment, the addition is made in one portion. According to another embodiment the addition is made gradually, e.g. dropwise or in several portions.

According to a still further embodiment, the nitroethane is added to the mixture of compound (II) and the base. The nitroethane can be added in one portion or gradually, e.g. dropwise or in several portions.

After step (i), a work-up of the reaction mixture can be carried out or the reaction mixture can be forwarded to the next step without any work-up.

According to one embodiment the work-up is carried out. It can be done by procedures known to the person skilled in the art. For example, after completion of the reaction an aqueous acidic solution is added to adjust the pH of the mixture to 4-7, preferably to pH 5-6. Sometimes it is practicable to add the reaction mixture to aqueous acidic solution after completion of the reaction.

The organic phases are then optionally washed with water. Further, it may be appropriate to wash the organic phases with acidic or basic aqueous solution instead or in addition to washing with water.

Then the solvent is removed from the separated organic phases.

The so-obtained raw product can be directly used in the next process step. According to one specific embodiment, it is first extracted from the organic phases in an aqueous alkaline solution and then used in the next process step, i.e. in step (ii) of the inventive process as described herein.

However, the raw product can also be further purified as generally known to the skilled person. If this is deemed appropriate, the reaction mixture is extracted with a suitable organic solvent (for example aromatic hydrocarbons such as toluene and xylenes) and the residue is, if appropriate, purified by recrystallization, distillation and/or chromatography.

According to a further embodiment the reaction mixture is directly forwarded from step (i) to the next step, i.e. step (ii) as described herein, without any work-up. According to this embodiment, steps (i) and (ii) are carried out in situ.

By means of the inventive process, the compounds of formula (I) can be prepared in surprisingly high yields. Preferably, the yields of step (ii) are at least 70%, more preferably at least 75 %, even more preferred at least 80%, even more preferred at least 90%.

The compound of formula (I) is also subject matter of the present invention.

According to step (ii) of the process, compounds (I) are converted into compounds of formula (III) 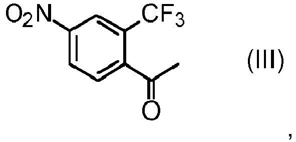 in the presence of an oxidant.

The oxidant is preferably selected from permanganates, e.g. potassium permanganate (KMnO₄); perborates e.g. sodium perborate; percarbonates e.g. sodium percarbonate; peroxides e.g. hydrogen peroxide, carbamide peroxide and t-butyl hydroperoxide; hypochlorites e.g. sodium hypochlorite; persulfates e.g. ammonium persulfate or potassium hydrogen persulfate (e.g. Oxone™); peroxy acids, e.g. perchloric acid; oxygen, air and the like.

The oxidant is more preferably selected from permanganates, peroxides, persulfates, oxygen and air; most preferably from permanganates, peroxides and persulfates.

According to one embodiment, the oxidant is selected from permanganates. Preferably it is KMnO₄.

According to another embodiment, the oxidant is selected from perborates. Preferably it is sodium perborate.

According to another embodiment, the oxidant is selected from percarbonates. Preferably it is sodium percarbonate.

According to another embodiment, the oxidant is selected from peroxides. Preferably it is selected from hydrogen peroxide, carbamide peroxide and t-butyl hydroperoxide. According to one specific embodiment it is hydrogen peroxide. According to another specific embodiment it is t-butyl hydroperoxide.

According to another embodiment, the oxidant is selected from hypochlorites, preferably sodium hypochlorite.

According to another embodiment, the oxidant is selected from persulfates. Preferably it is selected from ammonium persulfate or potassium hydrogen persulfate. According to one specific embodiment it is ammonium persulfate. According to another specific embodiment it is potassium hydrogen persulfate.

According to another embodiment, the oxidant is selected from peroxy acids. Preferably it is perchloric acid.

According to another embodiment, the oxidant is oxygen.

According to another embodiment, the oxidant is air.

The molar ratio of the oxidant to compound (I) is generally in the range from 6:1 to 1:1, preferably from 4:1 to 1:1, more preferably from 3:1 to 1:1, most preferably from 2:1 to 1:1. The molar ratio of the oxidant to compound (II) can specifically be in the range from 1.5:1 to 1:1, more specifically from 1.3:1 to 1:1, most specifically from 1.1 to 1 to 1:1.

The process step (ii) can be arried out in the presence of a base. According to one embodiment, the reaction is carried out without the base. According to another embodiment, the reaction is carried out in the presence of the base. The base used in step (ii) can be an inorganic base or an organic base.

According to one embodiment the base is an inorganic base. Suitable inorganic bases are hydroxides, carbonates, hydrocarbonates, phosphates and hydrophosphates of alkali or earth alkali metals such as NaOH, KOH, Na₂CO₃, K₂CO₃, NaHCO₃, KHCO₃, Na₃PO₄, K₃PO₄, Na₂HPO₄, K₂HPO₄, NaH₂PO₄, KH₂PO₄; NH₃ or an aqueous solution thereof (NH₄OH); or any mixtures thereof.

According to one embodiment, the bases are selected from hydroxides such as NaOH or KOH. According to one specific embodiment, the inorganic base is NaOH. According to another specific embodiment, the inorganic base is KOH.

According to another embodiment, the bases are selected from carbonates, such as Na₂CO₃ or K₂CO₃; or hydrocatbonates such as NaHCO₃ or KHCO₃. According to one specific embodiment, the inorganic base is Na₂CO₃. According to another specific embodiment, the inorganic base is K₂CO₃. According to another specific embodiment, the inorganic base is NaHCO₃. According to another specific embodiment, the inorganic base is KHCO₃.

According to another embodiment, the bases are selected from phosphates, such as Na₃PO₄ or K₃PO₄; or hydrophosphates such as Na₂HPO₄, K₂HPO₄, NaH₂PO₄, KH₂PO₄. According to one specific embodiment, the inorganic base is Na₃PO₄. According to another specific embodiment, the inorganic base is K₃PO₄. According to another specific embodiment, the inorganic base is Na₂HPO₄. According to another specific embodiment, the inorganic base is K₂HPO₄. According to another specific embodiment, the inorganic base is NaH₂PO₄. According to another specific embodiment, the inorganic base is KH₂PO₄.

According to another specific embodiment, the inorganic base is NH₃ or an aqueous solution thereof (NH₄OH). According to one embodiment, the inorganic base is NH₃. According to another embodiment, the inorganic base is NH₄OH.

The base can be used in a gaseous form, solid form, e.g. solid pellets, flakes, micropills or powder, or as a solution, e.g. as aqueous solution.

According to another embodiment the base is an organic base. Examples of suitable organic bases are alkoxides, tertiary amines, quaternary ammonium salts, amidines, guanidine derivatives, pyridine, substituted pyridines, bicyclic amines or any mixture thereof.

According to one specific embodiment the organic base is selected from alkoxides. Examples of suitable alkoxides are (C₁-C₁₀)-alkyl-alkoxydes, such as NaOCH₃, KOCH₃, NaOC₂H₅, KOC₂H₅, NaOC₃H₇, KOC₃H₇, NaOCH(CH₃)₂, KOCH(CH₃)₂, NaOC(CH₃)₃, KOC(CH₃)₃, sodium *tert-*amylate, potassium *tert*-amylate, sodium 3,7-dimethyl-3-octylate, potassium 3,7-dimethyl-3-octylate and the like. Preferable alkoxide bases are selected from C₁-C₄-alkyl-alkoxydes, such as NaOCH₃, KOCH₃, NaOC₂H₅, KOC₂H₅, NaOC₃H₇, KOC₃H₇, NaOCH(CH₃)₂, KOCH(CH₃)₂, NaOC(CH₃)₃a, KOC(CH₃)₃. Most preferable alkoxides are NaOCH₃ and KOC(CH₃)₃.

According to another specific embodiment the organic base is selected from tertiary amines. Examples of suitable tertiary amines are tri-(C₁-C₆)-alkylamines such as trimethylamine, triethylamine, tributylamine and N,N-diisopropylethylamine; di-(C₁-C₆)-alkyl-phenylamines such as N,N-dimethylaniline and N,N-diethylaniline; N-methyl imidazole, N,N-dimethylaminopyridine and the like. Preferable tertiary amine bases are selected from trimethylamine, triethylamine, tributylamine and N,N-diisopropylethylamine.

According to another specific embodiment the organic base is selected from quaternary ammonium salts. Example of a suitable quaternary ammonium salt is tetrabutylammonium fluoride (TBAF).

According to another specific embodiment the organic base is selected from amidines. Examples of suitable amidines are 1,8-diazabicyclo[5.4.0]undec-7-en (DBU) and 1,5-diazabicyclo-[4.3.0]non-5-ene (DBN).

According to another specific embodiment the organic base is selected from guanidine derivatives. Examples of suitable guanidine derivatives tetramethyl guanidine (TMG), (1,5,7-triazabicyclo[4.4.0]dec-5-ene) (TBD) and the like.

According to another specific embodiment the organic base is selected from pyridine and substituted pyridines. Pyridine is preferred. Examples of suitable substituted pyridines are collidine, lutidines, picolines, N,N-dimethylaminopyridine and the like.

According to another specific embodiment the organic base is selected from bicyclic amines. Example of suitable bicyclic amines is 1,4-diazabicyclo[2.2.2]octane (DABCO) and the like.

The molar ratio of the base to compound (I) is generally in the range from 5:1 to 0.5:1, preferably from 4:1 to 1:1, more preferably from 3:1 to 1:1, most preferably from 2:1 to 1:1.

In the step (ii) a catalyst may be added to the reaction mixture.

Examples of suitable catalysts are Cu, Fe, Mn or Co catalysts.

According to one embodiment, the catalyst is a Cu catalyst, preferably Cu powder, Cu(I)Cl, Cu(II)Cl₂ or CuSO₄•5H₂0.

According to another embodiment, the catalyst is a Fe catalyst, preferably FeCl₃.

According to another embodiment, the catalyst is a Mn catalyst, preferably KMnO₄, MnCl₂•4 H₂O, MnO₂ and MnSO₄•H₂O.

According to another embodiment, the catalyst is a Co catalyst, preferably CoBr₂, CoCl₂•6H₂O, Co(OAc)₂, Co(NO₃)₂•6H₂O.

The catalyst is preferably added in NH₄OH.

Preferably, the catalyst is employed if H₂O₂, oxygen or air are used as the oxidant.

In one specific embodiment, Cu powder, preferably in NH₄OH, is added to the reaction mixture as a catalyst. Preferably, it is added when H₂O₂ or air is used as the oxidant.

In one specific embodiment, Cu(I)Cl, preferably in NH₄OH, is added to the reaction mixture as a catalyst. Preferably, it is added when H₂O₂ or air is used as the oxidant.

In another specific embodiment Cu(II)Cl₂ preferably in NH₄OH, is added to the reaction mixture as a catalyst. Preferably, it is added when H₂O₂ or air is used as the oxidant.

In another specific embodiment CuSO₄•5H₂0, preferably in NH₄OH is added to the reaction mixture as a catalyst. Preferably, it is added when H₂O₂ or air is used as the oxidant.

In another specific embodiment FeCl₃, preferably in/ NH₄OH, is added to the reaction mixture as a catalyst. Preferably, it is added when H₂O₂ or air is used as the oxidant.

In another specific embodiment KMnO₄, preferably in NH₄OH, is added to the reaction mixture as a catalyst. Preferably, it is added when H₂O₂ or air is used as the oxidant.

In another specific embodiment MnCl₂•4H₂O, preferably in NH₄OH, is added to the reaction mixture as a catalyst. Preferably, it is added when H₂O₂ or air is used as the oxidant.

In another specific embodiment MnO₂, preferably in NH₄OH, is added to the reaction mixture as a catalyst. Preferably, it is added when H₂O₂ or air is used as the oxidant.

In another specific embodiment MnSO₄•H₂O, preferably in NH₄OH, is added to the reaction mixture as a catalyst. Preferably, it is added when H₂O₂ or air is used as the oxidant.

In another specific embodiment CoBr₂, preferably in NH₄OH, is added to the reaction mixture as a catalyst. Preferably, it is added when H₂O₂ or air is used as the oxidant.

In another specific embodiment CoCl₂•6H₂O, preferably in NH₄OH, is added to the reaction mixture as a catalyst. Preferably, it is added when H₂O₂ or air is used as the oxidant.

In another specific embodiment Co(OAc)₂, preferably in NH₄OH, is added to the reaction mixture as a catalyst. Preferably, it is added when H₂O₂ or air is used as the oxidant.

In another specific embodiment Co(NO₃)₂•6H₂O, preferably in NH₄OH, is added to the reaction mixture as a catalyst. Preferably, it is added when H₂O₂ or air is used as the oxidant.

The molar ratio of the catalyst to compound (I) is generally from 0.0001:1 to 0.5:1, preferably from 0.005:1 to 0.5:1, more preferably from 0.075:1 to 0.4:1, most preferably from 0.01:1 to 0.3:1, especially preferably from 0.05:1 to 0.1:1

The step (ii) according to the present invention can be carried out with or without a solvent.

According one embodiment, the reaction is carried out without a solvent.

According another embodiment, the reaction is carried out in an inert organic solvent. Examples of suitable inert organic solvents are aromatic hydrocarbons, such as benzene, toluene, ethylbenzene, cymene, mesitylene, o-xylene, m-xylene or p-xylene; aliphatic hydrocarbons such as n-pentane, n-hexane, n-heptane, n-octane, n-nonane, n-decane and their isomers, petroleum ethers, cyclohexane, methylcyclohexane; acetone, acetonitrile, alcohols, such as methanol, ethanol, 1-propanol, 2-propanol, 1-butanol and 2-butanol; esters such as alkyl acetates, e.g. ethyl acetate, butyl acetate, i-propyl acetate; dimethylformamide (DMF), N-nethyl-2-pyrrolidon (NMP), tetrahydrofuran (THF), dimethylsulfoxid (DMSO) or any mixture thereof. The solvent is preferably selected from acetone, acetonitrile, methanol, ethanol, ethyl acetate, butyl acetate, DMF, DMSO or any mixtures thereof. According to another specific embodiment, the solvent is ethyl acetate. According to another specific embodiment, the solvent is butyl acetate. According to one specific embodiment, the solvent is DMF. According to another specific embodiment, the solvent is DMSO.

The reagents in step (ii) can be added at ambient temperature or under cooling. According to one embodiment the reagents are added at ambient temperature. According to another embodiment the addition of reagents is carried out at a temperature between 0 and 20°C, preferably between 5 and 15°C. After the reagents have been added the reaction temperature is preferably held at ambient temperature. The temperature can be also elevated to 30-50°C.

The order of adding the reagents to the reaction mixture is variable.

According to one embodiment, the oxidant and the base are added to the compound (I). The oxidant and the base can be added separately or in form of a mixture thereof. According to one specific embodiment, the oxidant and the base are added separately to the compound (I). They can be added subsequently or simultaneously. According to another embodiment, the oxidant and the base are mixed together and then added to the compound (I). The addition of the oxidant and the base, in from of single compounds or in form of the mixture thereof, can be done in one portion or gradually, e.g. dropwise or in several portions. According to one embodiment, the addition is made in one portion. According to another embodiment the addition is made gradually, e.g. dropwise or in several portions.

According to one embodiment, the oxidant and the compound (I) are added to the base. The oxidant and the compound (I) can be added separately or in form of a mixture thereof. According to one specific embodiment, the oxidant and the compound (I) are added separately to the base. They can be added subsequently or simultaneously. According to another embodiment, the oxidant and the compound (I) are mixed together and then added to the base. The addition of the oxidant and the compound (I), in from of single compounds or in form of the mixture thereof, can be done in one portion or gradually, e.g. dropwise or in several portions. According to one embodiment, the addition is made in one portion. According to another embodiment the addition is made gradually, e.g. dropwise or in several portions. The base is preferably suspended or dissolved in sufficient solvent and is stirred during the addition of the reagents.

According to one embodiment, the base and the compound (I) are added to the oxidant. The base and the compound (I) can be added separately or in form of a mixture thereof. According to one specific embodiment, base and the compound (I) are added separately to the oxidant. They can be added subsequently or simultaneously. According to another embodiment, the base and the compound (I) are mixed together and then added to the oxidant. The addition of the base and the compound (I), in from of single compounds or in form of the mixture thereof, can be done in one portion or gradually, e.g. dropwise or in several portions. According to one embodiment, the addition is made in one portion. According to another embodiment the addition is made gradually, e.g. dropwise or in several portions.

According to a further embodiment, the oxidant is added to the mixture of compound (I) and the base. The oxidant can be added in one portion or gradually, e.g. dropwise or in several portions. According to one embodiment, the addition is made in one portion. According to another embodiment the addition is made gradually, e.g. dropwise or in several portions.

According to a further embodiment, the base is added to the mixture of compound (I) and oxidant. The mixture of compound (I) and oxidant can be prepared by adding compound (I) to oxidant or vice versa, by adding oxidant to the compound (I). Adding of oxidant to compound (I) is preferred. The base can be added in one portion or gradually. According to one embodiment, the addition is made in one portion. According to another embodiment the addition is made gradually, e.g. dropwise or in several portions.

The step (ii) may be conducted in a single phase or in a biphasic system.

According to one embodiment, the reaction is carried out in a single phase aqueous alkaline solution system or in a single phase aqueous alkaline solution and water-miscible organic solvent system.

According to another embodiment, the reaction is carried out in a biphasic system of an aqueous alkaline solution and a water-immiscible organic solvent.

The aqueous alkaline solution may be an aqueous solution of an alkali or alkaline earth metal hydroxide, carbonate, bicarbonate, phosphate or hydrophosphate.

After step (ii), a work-up of the reaction mixture can be carried out by procedures known in a general manner to the person skilled in the art. For example, after completion of the reaction water is added. The organic phases are separated, washed with water and the solvent is removed from the separated organic phases. Further, it may be appropriate to wash the organic phases with acidic or basic aqueous solution instead or in addition to washing with water.

The so-obtained raw product can be directly used in the next process step, i.e. step (iii) of the inventive process. However, the raw product can also be further worked up and/or purified as generally known to the skilled person. If this is deemed appropriate, the reaction mixture is extracted with a suitable organic solvent (for example aromatic hydrocarbons such as toluene and xylenes) and the residue is, if appropriate, purified by recrystallization and/or chromatography.

By means of the inventive process, the compounds of formula (III) can be prepared in surprisingly high yields. Preferably, the yields of step (ii) are at least 80%, more preferably at least 85 %, even more preferred at least 90%, even more preferred at least 95%.

The ketones (V) obtained according to the inventive process can be used as reagent for the synthesis of an oxirane of the formula (O) that are useful intermediates for the synthesis of triazole active ingredients of formula (T), that are effective against phytopathogenic fungi.

In the following, a possible synthesis route to such fungicides using said intermediates (V) is described:
Consequently, the present invention also relates to a process for the preparation of triazole compounds of the formula (T) 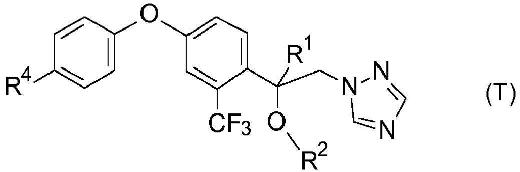 wherein
   - R¹: is C₁-C₆-alkyl or C₃-C₈-cycloalkyl;
   - R²: is hydrogen, C₁-C₆-alkyl, C₂-C₆-alkenyl, C₂-C₆-alkynyl, C₃-C₈-cycloalkyl, C₃-C₈-cycloalkyl-C₁-C₆-alkyl, phenyl, phenyl-C₁-C₄-alkyl, phenyl-C₂-C₄-alkenyl or phenyl-C₂-C₄-alkynyl;
   wherein the aliphatic moieties of R² are not further substituted or do carry one, two, three or up to the maximum possible number of identical or different groups R^{2a} which independently are selected from:
   R^{2a} halogen, OH, CN, nitro, C₁-C₄-alkoxy, C₃-C₈-cycloalkyl, C₃-C₈-halocycloalkyl and C₁-C₄-halogenalkoxy;
   wherein the cycloalkyl and/or phenyl moieties of R² are not further substituted or do carry one, two, three, four, five or up to the maximum number of identical or different groups R^{2b} which independently are selected from:
   R^{2b} halogen, OH, CN, nitro, C₁-C₄-alkyl, C₁-C₄-alkoxy, C₁-C₄-halogenalkyl, C₃-C₈-cycloalkyl, C₃-C₈-halocycloalkyl and C₁-C₄-halogenalkoxy;
   - R⁴: is halogen, preferably F or Cl
comprising the following steps:
   (iv) reacting the ketone of the formula (V) 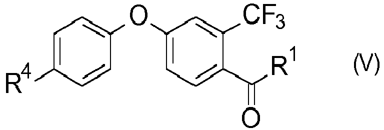 wherein the variables have the following meaning:
      - R¹: is C₁-C₆-alkyl or C₃-C₈-cycloalkyl
      - R⁴: is as defined for compounds of formula (T)
      to oxiranes (O); 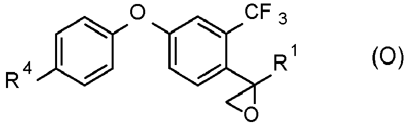 R¹ and R⁴ are as defined for compounds of formula (T)
   (v) reacting the oxirane (O) with 1H-1,2,4-triazole in the presence of a base to obtain compounds (T), wherein R² is hydrogen (compounds T-1); and, for obtaining compounds wherein R² is different from hydrogen:
   (vi) derivatizing the compound of formula (T-1) of step (v) under basic conditions with R²-LG, wherein LG is a nucleophilically replaceable leaving group; to result in compounds (T-2).

According to one embodiment, R² is selected from C₁-C₆-alkyl, C₂-C₆-alkenyl, C₂-C₆-alkynyl, C₃-C₈-cycloalkyl, C₃-C₈-cycloalkyl-C₁-C₄-alkyl, phenyl, phenyl-C₁-C₄-alkyl, phenyl-C₂-C₄-alkenyl and phenyl-C₂-C₄-alkynyl, wherein the R² are in each case unsubstituted or are substituted by R^{12a} and/or R^{12b} as defined and preferably defined herein.

According to a further embodiment, R² is C₁-C₆-alkyl, in particular C₁-C₄-alkyl, such as CH₃, C₂H₅, CH(CH₃)₂, CH₂CH₂CH₃, CH₂CH₂CH₂CH₃, CH₂CH(CH₃)₂. A further embodiment relates to compounds, wherein R² is C₁-C₆-alkyl, in particular C₁-C₄-alkyl, that is substituted by one, two or three or up to the maximum possible number of identical or different groups R^{12a}, as defined and preferably defined herein. According to still another embodiment, R² is C₃-C₈-cycloalkyl-C₁-C₆-alkyl, in particular C₃-C₆-cycloalkyl-C₁-C₄-alkyl. A further embodiment relates to compounds, wherein R² is C₃-C₈-cycloalkyl-C₁-C₆-alkyl, in particular C₃-C₆-cycloalkyl-C₁-C₄-alkyl, more particularly C₃-C₆-cycloalkyl-C₁-C₂-alkyl, that is substituted by one, two or three or up to the maximum possible number of identical or different groups R^{12a} in the alkyl moiety and/or substituted by one, two, three four or five or up to the maximum possible number of identical or different groups R^{12b} in the cycloalkyl moiety. R^{12a} and R^{12b} are in each case as defined and preferably defined herein.

According to another embodiment, R² is C₂-C₆-alkenyl, in particular C₂-C₄-alkenyl, such as CH₂CH=CH₂, CH₂C(CH₃)=CH₂ or CH₂CH=CHCH₃. A further embodiment relates to compounds, wherein R² is C₂-C₆-alkenyl, in particular C₂-C₄-alkenyl, that is substituted by one, two or three or up to the maximum possible number of identical or different groups R^{12a} as defined and preferably defined herein. According to a specific embodiment thereof, R² is C₂-C₆-haloalkenyl, in particular C₂-C₄-haloalkenyl, such as CH₂C(Cl)=CH₂ and CH₂C(H)=CHCl. According to still another embodiment, R² is C₂-C₆-alkynyl, in particular C₂-C₄-alkynyl, such as CH₂C≡CH or CH₂C≡CCH₃. A further embodiment relates to compounds, wherein R² is C₂-C₆-alkynyl, in particular C₂-C₄-alkynyl, that is substituted by one, two or three or up to the maximum possible number of identical or different groups R^{12a}, as defined and preferably defined herein.

According to still another embodiment, R² is C₃-C₈-cycloalkyl, in particular C₃-C₆-cycloalkyl, such as C₃H₅ (cyclopropyl), C₄H₇ (cyclobutyl), cyclopentyl or cyclohexyl. A further embodiment relates to compounds, wherein R² is C₃-C₈-cycloalkyl, in particular C₃-C₆-cycloalkyl, such as C₃H₅ (cyclopropyl) or C₄H₇ (cyclobutyl), that is substituted by one, two, three four or five or up to the maximum possible number of identical or different groups R^{12b} as defined and preferably defined herein. In a further embodiment of the invention, R² is selected from C₁-C₆-alkyl, C₂-C₆-alkenyl and C₂-C₆-alkynyl, wherein the R² are in each case unsubstituted or are substituted by R^{12a} and/or R^{12b} as defined and preferably defined herein. In each case, the substituents may also have the preferred meanings for the respective substituent as defined above.

R^{12a} according to the invention is preferably independently selected from F, Cl, OH, CN, C₁-C₂-alkoxy, cyclopropyl, 1-F-cyclopropyl, 1-Cl-cyclopropyl and C₁-C₂-halogenalkoxy.

R^{12b} according to the invention is preferably independently selected from F, Cl, OH, CN, nitro, CH₃, OCH₃, cyclopropyl, 1-F-cyclopropyl, 1-Cl-cyclopropyl and halogenmethoxy.

Specifically, the following compounds T.1 to T.7 can advantageously be prepared using the process according to the present invention:

| | |
|---|---|
| compound T.1 | 2-[4-(4-chlorophenoxy)-2-(trifluoromethyl)phenyl]-1-(1,2,4-triazol-1-yl)propan-2-ol; |
| compound T.2 | 1-[4-(4-chlorophenoxy)-2-(trifluoromethyl)phenyl]-1-cyclopropyl-2-(1,2,4-triazol-1-yl)ethanol; |
| compound T.3 | 2-[4-(4-chlorophenoxy)-2-(trifluoromethyl)phenyl]-3-methyl-1-(1,2,4-triazol-1-yl)butan-2-ol; |
| compound T.4 | 2-[4-(4-chlorophenoxy)-2-(trifluoromethyl)phenyl]-1-(1,2,4-triazol-1-yl)butan-2-ol; |
| compound T.5 | 1-[2-[4-(4-chlorophenoxy)-2-(trifluoromethyl)phenyl]-2-methoxy-propyl]-1,2,4-triazole; |
| com pound T.6 | 1-[2-[4-(4-chlorophenoxy)-2-(trifluoromethyl)phenyl]-2-cyclopropyl-2-methoxyethyl]-1,2,4-triazole; |
| compound T.7 | 1-[2-[4-(4-chlorophenoxy)-2-(trifluoromethyl)phenyl]-2-methoxy-butyl]1,2,4-triazole; |

Compounds (T) comprise chiral centers and they are generally obtained in the form of racemates. The R- and S-enantiomers of the compounds can be separated and isolated in pure form with methods known by the skilled person, e.g. by using chiral HPLC. Furthermore, components I can be present in different crystal modifications, which may differ in biological activity. The compounds may be present in various crystal modifications. They are likewise provided by the present invention.

In process step (iv), for obtaining an oxirane from the keto group, compound (V) is preferably reacted with a trimethylsulf(ox)onium halide (CH₃)₃S⁺(O)Hal⁻ (S1) or trimethylsulfonium methylsulfate (CH₃)₃S⁺ CH₃SO₄⁻ (S2).

According to one embodiment, in the process step (iv), the ketone (V) is reacted with trimethylsulfonium methylsulfate of the formula (S2) (CH₃)₃S⁺ CH₃SO₄⁻, preferably in aqueous solution in the presence of a base.

Step (iv) for the preparation of oxiranes (O) particularly is as follows:
(iv) reacting an oxo compound of the formula (V) with trimethylsulfonium methylsulfate of the formula (S2)

   S2 (CH₃)₃S⁺ CH₃SO₄⁻

in aqueous solution in the presence of a base, wherein the variables R¹, R⁴ are defined as given and preferably described herein for compounds (T).

In this process step (iv) using trimethylsulfonium methylsulfate of the formula (S2), preferably, 1 to 4 equivalents, in particular 1.2 to 3.5 eq, more specifically 1.5 to 3.3 eq, of water in relation to one equivalent of compound (V) are used. It may be favorable, if more than 1.5 eq of water, in particular more than 1.5 eq of water to 4 eq of water, more specifically more than 1.5 eq to 3.5 eq of water, even more particularly more than 1.5 eq water to 2.5 eq water per mole of compound (V) are used. In particular the ratios of 1.6 to 3.8, more specifically 1.7 to 3.3 eq, more specifically 1.8 to 2.8 eq or 1.9 to 2.5 of water per mole of compound (V) may be favorable according to the present invention. According to a further embodiment, more than 1.5 eq of water, in particular more than 2 eq of water, more specifically more than 2.5 eq of water per mole of compound (V) are used. In particular, 1.6 to 5, more specifically 1.7 to 4 eq, more specifically 1.8 to 3.5 eq of water per mole of compound (V) may be favorable according to the present invention.

The reagent (S2) is preferably used in an amount of 1.1 to 2.5, in particular 1.2 to 2, more specifically 1.3 to 1.6 equivalents of (S2) per 1 equivalent (mole) of compound (V).

In general, the reagent of formula (S2) can be prepared from dimethylsulfide and dimethylsulfate. According to one embodiment, reagent (S2) is prepared in-situ by adding dimethylsulfate to the reaction mixture containing dimethylsulfide. According to a further embodiment, either dimethylsulfide or dimethylsulfate is charged first and the other reagent is then added, wherein it may be preferred to add dimethylsulfide to a reaction mixture containing dimethylsulfate. The dimethylsulfide is usually used in excess. In particular, dimethylsulfide is generally used in amounts so that the reagent (S2) is sufficiently formed during the reaction. The molar ratio between dimethylsulfide and dimethylsulfate for the formation of the reagent (S2) is 1:1 to 2:1. Preferably, the molar ratio between dimethylsulfide and dimethylsulfate is 1:1 to 1.5:1, more preferably 1:1 to 1.4:1. It may be also preferred to use 1 to 1.3, in particular 1 to 1.25, more specifically 1 to 1.1 eq dimethylsulfide in relation to one equivalent of dimethylsulfate.

It is preferred to use as reagent (S2) an aqueous solution of trimethylsulfonium methylsulfate containing 33 to 37 wt%, preferably 34 to 36 wt%, more specifically 34 to 35.3 wt%, also more specifically 34.3 to 35.9 wt%, of trimethylsulfonium kation.

In particular, the reagent (S2) solution contains 33 to 37 wt%, preferably 34 to 36 wt%, more specifically 34 to 35.3 wt%, also more specifically 34.3 to 35.9 wt%, of trimethylsulfonium kation. Accordingly, the amount of trimethylsulfonium-methylsulfate in the reagent, measured as summation of trimethsulfonium-cation and methylsulfate-anion, is about 80 to 90 wt%, preferably about 83 to 88 wt-%, more specifically about 83 to 86 wt-%. The quantification can be, for example, accomplished by means of quantitative NMR-spectroscopie.

The viscosity of the aqueous reagent (S2) solution is comparatively low. The solutions are stable at room temperature, in particular at 25°C, and can be stored over a longer time. In particular, the reagent solution does not crystallize out during storage over a longer time, such as several weeks, e.g. up to 12 weeks, at temperatures of 10 to 25°C.

The reagent can be prepared by adding dimethylsulfate to water and dimethylsulfide. Dimethylsulfide is normally used in excess, generally 2 to 8, more preferably 4 to 6, more specifically 4.5 to 5.5, equivalents.

In the preparation of the aqueous solution of reagent (S2), in particular 0.8 to 2.2 eq, more preferably 0.9 to 1.2 eq, water in relation to the dimethylsulfate are used. It may also be preferred if in the preparation of the aqueous solution of reagent (S2), preferably 1.3 to 2.2 eq, more preferably 1.45 to 2.0 eq, water in relation to the dimethylsulfate are used.

Preferably, the temperature of the reaction mixture when adding the dimethylsulfate is room temperature, in particular 25°C to 40°C.

The aqueous reagent separates as the lower phase and can be further used as such.

The use of the aqueous solution of the reagent (S2) has been proven very efficient also for up-scaled reaction conditions, since it is stable and since it contains a defined amount of reagent, so that reagent (S2) can be easily and precisely dosed to the reaction mixture.

Thus, it is a preferred embodiment, if the reagent (S2) is added as an aqueous solution of trimethylsulfonium methylsulfate containing 33 to 37 wt%, preferably 34 to 36 wt%, more specifically 34 to 35.3 wt%, also more specifically 34.3 to 35.9 wt% of trimethylsulfonium kation.

According to one embodiment of the inventive process, dimethylsulfide is also used as solvent in step (iv). According to a further embodiment, an additional solvent is used. In particular, an aprotic organic solvent is suitable, such as for example diethylether, methyl-tert-butylether, chlorobenzene, xylene or toluene.

The base that can be used in step (iv) is preferably selected from KOH and NaOH. In a preferred embodiment, KOH is used, preferably as solid pellets or flakes. It is preferred if at least 3 equivalents of base, preferably at least 3.2 eq, more specifically at least 3.4 eq per 1 equivalent of compound (V) are used. It may be preferred if the amount of base is 3 to 6 eq, more specifically 3 to 5 eq per mole of compound (V).

The reaction temperature when adding KOH in step (iv) is preferably held at a maximum of 60°C, more specifically at a maximum of 50°C. Generally, it is also preferred to have a reaction temperature when adding KOH of at least 20°C, in particular at least room temperature, in particular at least 25°C. In a further embodiment, the temperature is at least 30°C. It may be preferred if the temperature is at least 35°C or at least 45°C. The temperature of the reaction mixture can for example be held in these ranges by adding the KOH in portions.

The overall reaction temperature in step (iv) is preferably held at a maximum of 70°C, in particular at a maximum of 60°C, more preferably at a maximum of 50°C. Generally, it is also preferred to have a reaction temperature of at least 20°C, in particular at least room temperature, in particular at least 25°C. In a further embodiment, the temperature is at least 30°C. It may be preferred if the temperature is at least 35°C.

In case a work-up of the reaction mixture after step (iv) is suitable, it can be carried out by procedures known in a general manner to the person skilled in the art. It may be preferred if water is added to the reaction mixture after completion of step (iv) and the resulting mixture is heated while stirring dependent on the melting point of the organic content. The temperature during this heating is held preferably from 30°C to 70°C, more specifically 40°C to 60°C, even more specifically 50°C to 60°C. The organic phase may, for example, be separated and dissolved in a suitable solvent such as dimethyl formamide (DMF), N-methylpyrrolidone (NMP), dimethylsulfoxide (DMSO) or dimethylacetamide (DMAC). Dimethylsulfide, if still present, is preferably removed by distillation before or after the solvent addition. The reaction mixture may then be used directly for the next step or, if appropriate, further worked-up and/or purified by e.g. recrystallization and/or chromatography.

According to one further specific embodiment, in step (iv), the oxo compound of the formula (V) is reacted with dimethyl sulfide (CH₃)₂S and dimethylsulfate (CH₃)₂SO₄, forming the reagent (S2), trimethylsulfonium methylsulfate [(CH₃)₃S⁺ CH₃SO₄⁻], in aqueous solution in the presence of potassium hydroxide (KOH), wherein dimethyl sulfide and dimethyl sulfate are used in a molar ratio of 1:1 to 2:1, and wherein at most 10 weight-% organic solvent in relation to the amount of compound (V), are added. For details see also WO2016/005211. In this embodiment, the reagent of formula (S2) is formed from dimethylsulfide and dimethylsulfate. In particular, reagent (S2) is prepared in-situ. Either dimethylsulfide or dimethylsulfate is charged first and the other reagent is then added. It may be preferred to add dimethylsulfide to a reaction mixture containing dimethylsulfate.

The dimethylsulfide and dimethylsulfate are preferably used in such amounts that the reagent (S2) is present in the reaction mixture in an amount of 1.1 to 2.5, in particular 1.2 to 2, more specifically 1.3 to 1.6 equivalents of (S2) per 1 equivalent (mole) of compound (V).

Dimethylsulfide is used in amounts so that the reagent (S2) is sufficiently formed during the reaction. The molar ratio between dimethylsulfide and dimethylsulfate for the formation of the reagent (S2) is 1:1 to 2:1. Preferably, the molar ratio between dimethylsulfide and dimethylsulfate is 1:1 to 1.5:1, more preferably 1:1 to 1.4:1. It may be also preferred to use 1 to 1.3, in particular 1 to 1.25, more specifically 1 to 1.1 dimethylsulfide in relation to one equivalent of dimethylsulfate. This reaction step can be carried out with at most 10 weight-% of organic solvents in relation to the amount of compound (V) [amount of solvent: (amount of solvent + amount of compound IV)]. In particular, the reaction can be carried out using at most 8 weight-%, more specifically at most 5 weight-%, even more specifically at most 3 weight-%, of organic solvents in relation to the amount of compound (V). More specifically, in the reaction mixture, at most 2 weight-%, more specifically at most 1 weight-% of organic solvents in relation to the amount of compound (V) are added.

In a specific embodiment, in this step (iv) essentially no organic solvent is added. In particular, in step (iv) no organic solvent is added apart from the reagents used.

Organic solvents are liquid organic compounds that dilute the reagents without taking part in the reaction or catalyzing the reaction. The skilled person in the field of organic synthesis is familiar with "organic solvents" and it is clear to such skilled person what kind of solvents are "organic solvents". Examples for organic solvents are e.g. alcohols, nitrils and aromatic hydrocarbons. Alcohols are for example methanol, ethanol, propanol and butanol (e.g. tert-butanol). Aromatic hydrocarbons are for example toluene or xylenes. An example for nitrile is acetonitrile.

Reaction step (iv) is preferably carried out in aqueous solution. Preferably, water is used in an amount of 0.5 to 4 eq, in particular 0.9 to 4, in relation to one equivalent of compound (V). According to one embodiment, relatively low amounts of water, for example 0.5 to 0.95 eq, more specifically 0.6 to 0.94, even more specifically 0.7 to 0.93 eq in relation to one equivalent of compound (V), are used. It may also be advantageous to use 0.8 to 0.92 eq, more specifically 0.85 to 0.91, even more specifically 0.85 to 0.9 eq in relation to one equivalent of compound (V) in the inventive process. According to a further embodiment, 0.9 to 4 equivalents, more specifically 1 to 4, in particular 1.2 to 3.5 eq, more specifically 1.5 to 3.3 eq, of water in relation to one equivalent of compound (V) are used. It may be favorable, if more than 1.5 eq of water, in particular more than 1.5 eq of water to 4 eq of water, more specifically more than 1.5 eq to 3.5 eq of water, even more particularly more than 1.5 eq water to 2.5 eq water per mole of compound (V). In particular the ratios of 1.6 to 3.8, more specifically 1.7 to 3.3 eq, more specifically 1.8 to 2.8 eq or 1.9 to 2.5 of water per mole of compound (V) may be favorable according to the present invention. In one further particular embodiment, advantages can be achieved if the amounts of water used in step (iv) are 0.5 to 0.95 eq or more than 1.5 eq of water to 4 eq per mole of compound (V).

In step (iv), preferably KOH is used. It is preferred if at least 2 equivalents of base, more specifically at least 2.5 equivalents of base, even more specifically at least 3 equivalents of base per 1 equivalent of compound (V) are used. It may be preferably if at least 3.2 eq, more specifically at least 3.4 eq per 1 equivalent of compound (V) are used. Furthermore, it may be advantageous, if the amount of base is 2 to 6 eq, in particular 2.5 to 5.5 eq, more specifically 2.5 to 5 eq, even more specifically 3 to 5 eq per mole of compound (V).

KOH is in particular used in solid form, preferably as solid pellets, flakes, micropills and/or powder.

The base, in particular solid KOH, is in particular used such that the preferred range of water present in the reaction is kept. Then, some of the base is dissolved in the reaction solution and some is still present in solid form during the reaction.

The KOH can be added in one or more portions, for example 2 to 8 portions, to the reaction mixture. KOH can also be added in a continuous manner. Preferably, the KOH is added after compound (V) has been charged to the reaction vessel. However, the order may also be changed and the compound (V) is added to the reaction mixture already containing the KOH.

The order of adding the reagents to the reaction mixture is variable. In one embodiment, the base is added to the solution of compound (V) and solvent first and then reagent (S2) is added. According to another embodiment, the reagent (S2) is added first to the solution of compound (V) and then the base is added. According to a further embodiment, a solution of compound (V) and the reagent (S2) are added simultaneously to the base. In the latter embodiment, the base is preferably suspended in sufficient solvent and is stirred during the addition of the reagents.

In step (v) the oxiranes (O) can be further reacted to a triazole of formula (T) as defined above.

LG represents a nucleophilically replaceable leaving group such as halogen, alkylsulfonyl, alkylsulfonyloxy and arylsulfonyloxy, preferably chloro, bromo or iodo, particularly preferably bromo.

In one embodiment of the invention, in the process step (v) an inorganic base is used.

The inorganic base that may be used in step (v) is preferably selected from NaOH, KOH, Na₂CO₃ and K₂CO₃, more specifically from NaOH and KOH. According to one embodiment, NaOH is used. According to a further embodiment, KOH is used.

According to a specific embodiment, the sodium salt of 1H-1,2,4-triazole as a base is used, wherein said sodium salt is prepared using triazole and a base preferably selected from NaOH, NaH and Na-alcoholates. See also DE 3042302.

The amount of base used in step (v) is preferably equal to or less than 1 eq, in particular less than 1 eq, more preferably equal to or less than 0.8 eq, even more preferably equal to or less than 0.6 equivalents per 1 equivalent of compound (O). Also preferred are amounts of base being equal to or less than 0.4 equivalents, in particular equal to or less than 0.2 equivalents, specifically equal to or less than 0.1 eq per 1 equivalent of compound (O). Preferably, at least 0.1 eq, more preferably at least 0.2 equivalents, in particular at least 0.3, more specifically at least 0.4 eq base per 1 equivalent of compound (O) are used.

It may be favorable, if, in the synthesis of (T-1), less than 1 eq of base is used in relation to the compound (O). In specific embodiments thereof, NaOH is used as a base, preferably in an amount as given above, in particular in an amount of 0.1 to 0.55 eq in relation to the oxirane of formula (O).

In order to have preferably low reaction times, temperatures of at least 100 °C, more preferably at least 110 °C, in particular at least 120 °C are favorable. It is also an embodiment to reflux the reaction mixture. Preferably, the reaction temperature is not higher than 150°C, in particular not higher than 140°C. Specifically, a reaction temperature of 120°C to 140°C is used.

The amount of 1H-1,2,4-triazole used in step (v) generally is at least 1 eq per mole of oxirane (O). According to one embodiment, the 1H-1,2,4-triazole is used in excess in relation to the oxirane (O). Preferred are more than 1 eq to 2 eq, more preferably more than 1 eq to 1.8 eq, even more preferred more than 1 eq to 1.6 eq. Mostly for economic reason, it can be preferred to use at least 1.1 eq, specifically 1.15 eq, to 1.5 eq of triazole in relation to oxirane (O).

The solvent used in step (v) is preferably selected from dimethylformamide, dimethylacetamide, N-metylpyrrolidone. Most preferred is dimethylformamide.

According to one preferred embodiment, the compounds (T-1) resulting from step (v) are crystallized from a suitable solvent such as, for example toluene, an aliphatic alcohol, acetonitrile, ethyl acetate and/or cyclohexane, in particular toluene and/or an aliphatic alcohol.

Generally, one undesired side product in the synthesis of compounds (T-1) that may occur in undesired amounts is the symmetric triazole (Ts-1) that is formed together with the desired triazole of formula T-1, leading, consequently, to lower yields of the desired product.

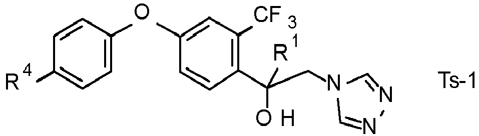

Consequently, according to one preferred embodiment of the invention, the products resulting from step (v) are crystallized from a suitable solvent. This step is called final work up step (v-1). Suitable solvents are, for example, selected from toluene, an aliphatic alcohol, acetonitrile, carbonic acid ester and cyclohexane, or any mixtures thereof, in particular from toluene, an aliphatic alcohol and carbonic acid ester and any mixture thereof.

In particular, the aliphatic alcohol is selected from methanol, ethanol, n-propanol, iso-propanol, n-butanol, isobutanol and any mixture thereof. In particular, the aliphatic alcohol is selected from methanol and ethanol and any mixture thereof.

Examples for suitable carbonic acid esters are n-butyl acetate or ethyl acetate and any mixture thereof.

Generally, for the crystallizing step, the reaction solvent, in particular dimethylformide as described above, is firstly evaporated in large part, preferably under reduced pressure. Preferably, at least 55% of the solvent, more preferably at least 60% of the sovent, more specifically at least 70% of the solvent are removed. Specifically, it may be preferred, if at least 80%, more specifically at least 90% of the solvent, such as DMF, are removed. The solvent can then be recycled to be used again in any previous process step, if necessary after it has been further rectificated before.

Then, water and the respective suitable solvent such as an ether, for example diethylether, diisopropylether, methyl-tert-butylether (MTBE), methylenechloride and /or tolulene, in particular toluene, are added. Also, ethyl acetate and/or n-butyl acetate can be appropriate as solvent. The product I is then preferably obtained by crystallization directly from the concentrated, e.g. toluene-reaction mixture. Also, preferred and suitable according to the invention is the change of solvent to e.g. methanol or ethanol (see above) for the crystallization of the products.

According to one embodiment, seed crystals are added for the crystallization step.

By using the crystallizing step, in particular when carrying out the process steps (v) the formation of the undesired symmetric triazole (Ts-1) as described above can be reduced to equal or less than 10%, more preferably equal or less than 8%, even more preferably equal or less than 5%, even more preferably equal or less than 2%.

Preferably, the ratio of isolated compound (T-1) to the symmetric triazole (Ts-1) is at least 20:1, more preferably at least 30:1, even more preferably 50:1, more specifically 70:1. In particular, the ratio of compound (T-1) to (Ts-1) is at least 30:1.

Also other methods of further reacting the oxiranes (O) to end products (T) can be carried out.

For example, the epoxide ring of compounds (O) may be cleaved by reaction with alcohols R²OH preferably under acidic conditions to result in compounds (VI):

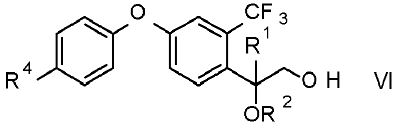

Thereafter, the resulting compounds (VI) are reacted with halogenating agents or sulfonating agents such as PBr₃, PCl₃ mesyl chloride, tosyl chloride or thionyl chloride, to obtain compounds (VII) wherein LG' is a nucleophilically replaceable leaving group such as halogen, alkylsulfonyl, alkylsulfonyloxy and arylsulfonyloxy, preferably chloro, bromo or iodo, particularly preferably bromo or alkylsulfonyl. Then compounds (VII) are reacted with 1H-1,2,4-triazole to obtain compounds (T) as known in the art and/or described above:

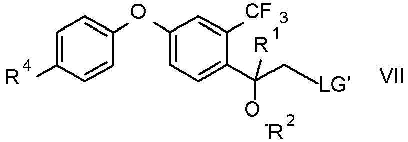

For obtaining compounds of formula T, wherein the alcohol group is derivatized into an ether group to result in compounds of formula T-2, wherein the variables are defined above, the following step can be carried out:
(vi) derivatizing the compound of formula (T-1) as defined in step (v) under basic conditions with R²-LG, wherein LG is a nucleophilically replaceable leaving group; to result in compounds (T-2).

LG represents a nucleophilically replaceable leaving group such as halogen, alkylsulfonyl, alkylsulfonyloxy and arylsulfonyloxy, preferably chloro, bromo or iodo, particularly preferably bromo. Preferably a base is used in step (vi) such as for example, NaH.

Suitable solvents are for example ethers, in particular cyclic ethers. Possible solvents are for example tetrahydrofuran (THF), 2-methyl-tetrahydrofuran (2-Me-THF), diethyl ether, TBME (tert-butyl methyl ether), CPME (cyclopentyl methyl ether), DME (1,2-dimethoxyethane) and 1,4-dioxane. Further solvents that may be suitable are, for example, diisopropyl ether, di-n-butyl ether and/or diglyme. Often, the use of THF or 2-methyl-THF is particularly suitable. Furthermore, it may also be suitable to use combinations of two or more different solvents, such as for example any combination of the solvents listed above or any one of the listed ethers with aliphatic hydrocarbons like n-hexane, heptane or aromatic hydrocarbons like toluene or xylenes.

The skilled person is familiar with the reaction in step (vi) and may vary the reaction conditions analogously to known syntheses.

In one embodiment, a triazole compound of the formula T is obtained by
(vi-a) reacting an oxirane of the formula (O) as defined herein with 1H-1,2,4-triazole and an inorganic base, wherein less than 1 equivalent of said base is used per 1 equivalent of compound (O), resulting in compounds of formula (T).

For obtaining compounds of formula (T-2), wherein the alcohol group is derivatized (resulting in "OR²", see above), the above derivatizing step can be carried out.

### Examples

The following examples further illustrate the present invention and do not restrict the invention in any manner.

### Example S1-1: Synthesis of 4-nitro-2-(trifluoromethyl)-1-(1-nitroethyl)-benzene (I)

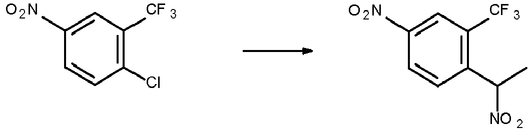

Potassium hydroxide pellets (37.3 g, 0.56 mol) were added to stirred DMSO (250 mL) at 25 °C. The reaction mixture was cooled to 18 °C and stirred for 10 min at this temperature. A mixture of nitroethane (20 g, 0.26 mol) and 4-nitro-2-(trifluoromethyl)-chlorobenzene (50 g, 0.22 mol) was added dropwise to the reaction mixture at a rate such that a temperature of 15-20 °C was maintained. Thereafter the reaction mixture was warmed to 25 °C and stirred for 3 hrs. Toluene (250 mL) was added to the reaction mixture and the reaction mixture was cooled to 15 °C. Aqueous hydrochloric acid (6M HCI 70.73 g, 0.38 mol) was added dropwise with rapid stirring at a rate such that temperature does not exceed beyond 20 °C (pH 1.6 - 1.8). Water (250 mL) was then added and the layers were separated. The organic layer was separated, washed with aqueous NaOH (2N, 35.7 mL, 0.07 mol) and then with water (100 mL). Thereafter, the organic layer was concentrated under vacuum to give 4-nitro-2-(trifluoromethyl)-1-(1-nitroethyl)-benzene (49 g, Yield - 79.5%; HPLC Purity - 95.3 %) as a light brown oil. Purity can be further increased to > 98% by triturating the product with 2 volumes of heptane.

### Example S1-2: Synthesis of 4-nitro-2-(trifluoromethyl)-1-(1-nitroethyl)-benzene (I)

1,8-Diazabicyclo[5.4.0]undec-7-ene (4 g, 0.026 mol) and nitroethane (2 g, 0.026 mol) were added to stirred toluene (6 mL) at 25 °C. The reaction mixture was cooled to 0 °C and stirred for 10 min at this temperature. Solution of 4-nitro-2-(trifluoromethyl)-chlorobenzene (2 g, 0.008 mol) in toluene (4 mL) was added to the reaction mixture dropwise at a rate such that a temperature of 0-5 °C was maintained. After addition was completed the reaction mixture was stirred for 3h at this temperature. Aqueous potassium bisulphate (1M 20 mL, 0.019 mol) was added to the reaction mixture. The temperature of the reaction mixture was raised to 25 °C, the organic layer was separated and concentrated under vacuum to get 4-nitro-2-(trifluoromethyl)-1-(1-nitroethyl)-benzene (2.2 g, Yield - 71,7%, HPLC Purity -73.68 %) as a light brown oil.

### Example S1-3: Synthesis of 4-nitro-2-(trifluoromethyl)-1-(1-nitroethyl)-benzene (I)

4-Nitro-2-(trifluoromethyl)-chlorobenzene (6.55 ml, 44.3 mmol, 1 eq) was slowly added to a mixture of tetramethylguanidine (TMG) (20 ml, 155 mmol, ca. 3.5 eq) and nitroethane (3.84 ml, 53.2 mmol, 1.2 eq) under nitrogen atmosphere at 0 °C. The reaction mixture was stirred for 2 h and then was quenched with toluene (100 ml) and water (100 ml). Thereafter, glacial acetic acid (6.66 ml, 115 mmol) was added to a separated aqueous layer and the mixture was stirred for 5 minutes. The aqueous layer was extracted two times with 100 ml of toluene. The combined organic layers were washed with a mixture of saturated aqueous NaHCO₃ solution (50 ml) and water (50 ml). The organic layer was then concentrated to give 4-nitro-2-(trifluoromethyl)-1-(1-nitroethyl)-benzene as a clear light brown oil (11.03 g, Yield - 92% with a purity of 97.6 area% and assay of 97.6% qHPLC).

### Example S2-1: Synthesis of 4-nitro-2-trifluoromethyl-acetophenone (III)

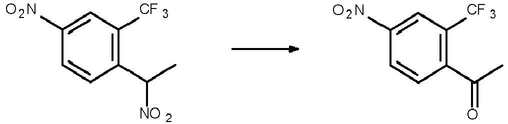

To a stirred solution of 4-nitro-2-(trifluoromethyl)-1-(1-nitroethyl)-benzene (67 g, 0.25 mol) in DMF (201 mL) at 25 °C was added a solution of copper (I) chloride (0.25 g, 0.0025 mol) in 30 % aqueous ammonia (18.7 g). To this solution 50 % H₂O₂ (34.5 g. 0.507 mol) was added dropwise at a rate such that a temperature of reaction was maintained between 25-30 °C. The reaction mixture was further stirred at 25 °C for 2h. Water (200 mL) and toluene (335 mL) were added to the reaction mixture. The organic layer was separated, washed with water and concentrated under vacuum to get 4-nitro-2-trifluoromethyl-acetophenone (55 g, Yield: 94.28 %, HPLC purity 96.22 %) as a light brown solid.

### Example S2-2: Synthesis of 4-nitro-2-trifluoromethyl-acetophenone (III)

To a rapidly stirred solution of 4-nitro-2-(trifluoromethyl)-1-(1-nitroethyl)-benzene (0.5g, 0.002 mol) in DMF (2.5 mL) at 25°C was added solution of copper sulfate pentahydrate (0.003 g, 0.012 mmol) in 30% aqueous ammonia (0.16 gm). To this mixture 50% H₂O₂ (0.25 g, 0.004 mol) was added dropwise at a rate such that a temperature of reaction was maintained between 25-30°C. The reaction mixture was further stirred for 3 h at 30°C. Thereafter, the reaction was quenched by charging 2.5 mL water and the product was extracted by methyl t-butyl ether. The organic layer was separated and concentrated to obtain 4-nitro-2-trifluoromethyl-acetophenone (0.35 gm, Yield 74.2%, HPLC purity 92.6%).

### Example S2-3: Synthesis of 4-nitro-2-trifluoromethyl-acetophenone (III)

To a rapidly stirred solution of 4-nitro-2-(trifluoromethyl)-1-(1-nitroethyl)-benzene (0.5g, 0.002 mol) in DMF (2.5 mL) at 25°C was added solution of copper powder (0.001 g, 0.02 mmol) in 30% aqueous ammonia (0.16 gm). To this mixture 50% H₂O₂ (0.25 g, 0.004 mol) was added dropwise at a rate such that a temperature of reaction was maintained between 25-30°C. The reaction mixture was further stirred for 3 h at 30°C. Thereafter, the reaction was quenched by charging 2.5 mL water and product was extracted by methyl t-butyl ether. The organic layer was separated and concentrated to obtain 4-nitro-2-trifluoromethyl-acetophenone (0.38 g, Yield 84.9%, HPLC purity 97.6%).

### Example S2-4: Synthesis of 4-nitro-2-trifluoromethyl-acetophenone (III)

To a rapidly stirred solution of 4-nitro-2-(trifluoromethyl)-1-(1-nitroethyl)-benzene (0.5g, 0.002 mol) in DMF (2.5 mL) at 25°C was added solution of ferric chloride (0.003 g, 0.018 mmol) in 30% aqueous ammonia (0.16 g). To this mixture added 50% H₂O₂ (0.25 g, 0.004 mol) was added dropwise at a rate such that a temperature of reaction was maintained between 25-30°C. The reaction mixture was further stirred for 3 h at 30°C. Thereafter, the reaction was quenched by charging 2.5 mL water and product was extracted by methyl t-butyl ether. The organic layer was separated and concentrated to obtain 4-nitro-2-trifluoromethyl-acetophenone (0.36 g, Yield 76.4%, HPLC purity 92.8%).

### Example S2-5: Synthesis of 4-nitro-2-trifluoromethyl-acetophenone (III)

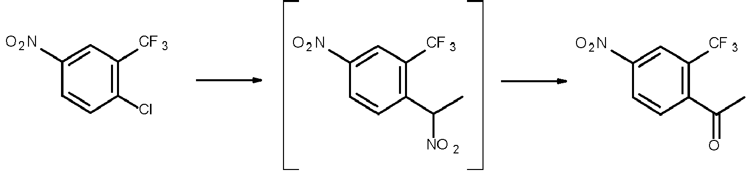

Potassium hydroxide pellets (3.73 g, 0.056 mol) were added to DMSO (25 mL) at 25 °C under stirring. The reaction mixture was cooled to 18 °C and stirred for 10 min at this temperature. A mixture of nitroethane (2 g, 0.026 mol) and 4-nitro-2-(trifluoromethyl)-chlorobenzene (5 g, 0.022 mol) was added dropwise to the reaction mixture at a rate such that a temperature of 15-20 °C was maintained. After addition reaction mixture was warmed to 25 °C and stirred for 3 h. Thereafter, concentrated sulfuric acid (2.65 g, 0.0265 mol) was added dropwise at a rate such that temperature does not exceed beyond 20 °C. A solution of copper (I) chloride (0.022 g, 0.0002 mol) in 30 % aqueous ammonia (1.63 g, 0.028 mol) was added to the reaction mixture followed by dropwise addition of 50 % H₂O₂ (3.0 g. 0.04 mol) at a rate such that a reaction mixture temperature was maintained at 25-30 °C. The reaction was further stirred for 2h at 25 °C. Water (20 mL) and toluene (25 mL) were added to the reaction mixture. The organic layer was separated, washed with water and concentrated under vacuum to get 4-nitro-2-trifluoromethylacetophenone (3.5 g, Overall yield: 57.15%, HPLC purity 84.42 %) as a light brown solid.

### Example S3: Synthesis of 4-(4-chlorophenyloxy)-2-trifluoromethyl-acetophenone

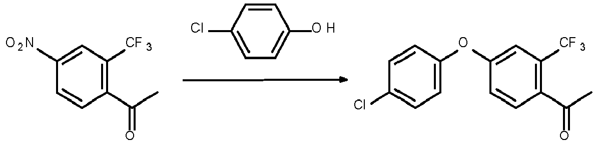

To a stirred solution of 4-nitro-2-trifluoromethyl-acetophenone (5 g, 0.02 mol) in DMF (225 mL) at 25 °C was added a potassium carbonate (2 g, 0.015 mol) and 4-chlorophenol (2.9 g, 0.022 mol). The reaction mixture was heated to 125°C and stirred for 3h at this temperature. Thereafter, the reaction mixture cooled down to 25 °C. Water (25 mL) and toluene (25 mL) were added. The organic layer was separated, washed with aqueous NaOH (10 mL, 0.02 mol), then with water (15 mL) and concentrated under vacuum to compound give 4-(4-chlorophenyloxy)-2-trifluoromethyl-acetophenone (6 g, Yield:86.37 % HPLC purity: 96.88 %) as a dark brown oil.

### Example S4: Synthesis of 2-[4-(4-chlorophenoxy)-2-(trifluoromethyl)phenyl]-2-methyl-oxirane

1-[4-(4-chlorophenoxy)-2-(trifluoromethyl)phenyl]ethanone (0.128 mole) dissolved in 55.4 g dimethylsulfide were provided at 22°C. 25.4 g KOH pellets, 85 wt-% (0.385 mole) were added while stirring heavily. Then, 42.1 g aqueous trimethylsulfonium-methylsulfate (85.6 wt-%, prepared according to Example A 1) were added. This led to an increase of temperature of about 2 to 3°C. Then, it was continued stirring for 8 h at 38°C. A sample of the reaction mixture showed full conversion of the ketone (HPLC). After that, 199 g 20 wt-% NaCl solution was added at 30°C. After separation of the aqueous phase, the dimethylsulfide solution was concentrated by means of distillation of the solvent at a temperature of up to 90°C. 49.7 g of about 82 wt-% ( quant. H PLC) 2-[4-(4-chlorophenoxy)-2-(trifluoromethyl)phenyl]-2-methyl-oxirane were obtained; yield about 97 %.

### Example S5: (Preparation 2-[4-(4-chlorophenoxy)-2-(trifluoromethyl)phenyl]-1-(1,2,4-triazol-1-yl)propan-2-ol

109 g (51.3 wt-% in DMF; 0.1701 mole) 2-[4-(4-chlorophenoxy)-2-(trifluoromethyl)phenyl]-2-methyl-oxirane were diluted with 105.6 g DMF at room temperature. 15.6 g (98 wt-%; 0.221 mole) of 1,2,4-triazole and 3.47 g (0.085 mole) NaOH flakes were added under stirring. The reaction mixture was heated to 125 - 126°C and then stirred for 5 h in total at this temperature. A HPLC-sample showed complete conversion to the desired product (ratio triazol-1-yl/triazol-4-yl about 10:1). About 93% of the DMF was evaporated at 125°C / 300-60 mbar. To the concentrated reaction mixture, 150 g butyl acetate and 92.3 g water were added and the mixture stirred over 10 minutes. Then, the aqueous phase was separated at 80°C.

The organic phase was concentrated at 85°C / 400-130 mbar by 50% (distillate of 117.6 g butyl acetate). The solution was cooled to 60°C and seeded with product and stirred at this temperature over 30 minutes so that the product crystallized slowly. Further cooling to 0°C with a rate of 7.5°K/h followed by suction filtration of the product, washing with 42.8 g n-butyl acetate at 0°C and drying in a drying cabinet at 55°C / 15 mbar led to 52.1 g of product (78.1% of the theory).

## Claims

1. A process for the preparation of the compound of formula (V) 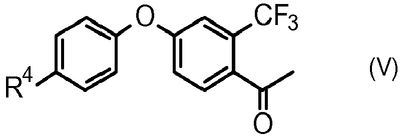 wherein
R⁴ is halogen;
comprising the following step:
(iii) reacting a compound of formula (III) 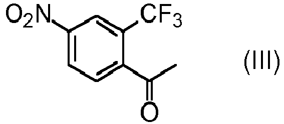
with a phenol derivative of formula (IV) 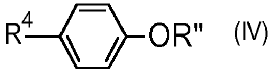 wherein
R" is hydrogen or an alkali metal ion;
R⁴ is as defined for compounds of formula (V);
in the presence of a base.

2. The process of claim 1, wherein the base is an inorganic base selected from hydroxides, carbonates, hydrocarbonates, phosphates and hydrophosphates of alkali or earth alkali metals or any mixtures thereof.

3. The process of any of claims 1 to 3, wherein the base is selected from NaOH, KOH, CsOH, Na₂CO₃, K₂CO₃, Cs₂CO₃, NaHCO₃, KHCO₃, Na₃PO₄, K₃PO₄, Na₂HPO₄, K₂HPO₄, NaH₂PO₄, KH₂PO₄.

4. The process of claim 1, wherein the base is a organic base selected from selected from alkoxides, acetates, tertiary amines, pyridine, substituted pyridines, or any mixtures thereof.

5. The process of any of claims 1 to 4, wherein the base is used in an amount of 0.5 to 2 mole equivalents per 1 mole of compound (III).

6. The process of any of claims 1 to 5, wherein the base is used in an amount of 0.5 to 1.1 mole equivalents per 1 mole of compound (III).

7. The process of any of claims 1 to 6, wherein the reaction is carried out in the presence of a catalyst.

8. The process of claim 7, wherein the catalyst is selected from Cu(I), Cu(II) or Fe(III) catalysts.

9. The process of claim 7 or 8, wherein the catalyst is selected from Cu(I)Cl, Cu(II)Cl₂, CuSO₄•5H₂0, Cu(OAc)₂ and FeCl₃.

10. The process of any of claims 1 to 9, wherein the reaction is carried out in a solvent selected from dimethyl formamide (DMF), N-methyl pyrrolidone (NMP), dimethyl imidazolidinone (DMI), toluene, o-xylene, m-xylene, p-xylene, dimethylactamide (DMA), dimethylsulfoxide (DMSO) and any mixtures thereof.

11. The process of any of claims 1 to 10, wherein the reaction is carried out in a solvent selected from DMF, DMSO or any mixture thereof.

12. The process of any of claims 1 to 11, wherein compound of formula (II) is used in an amount of 1 to 1.3 mole equivalents per 1 mole of compound (III).

13. The process of any of claims 1 to 12, wherein the reaction is carried out at a temperature from 80 to 160 °C.

14. The process of any of claims 1 to 13, wherein the reaction is carried out at a temperature from 100 to 140 °C.

15. A process for the preparation of triazole compounds of the formula (T) 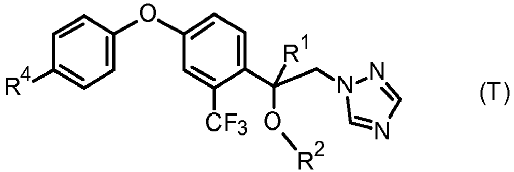 wherein R¹ is C₁-C₆-alkyl or C₃-C₈-cycloalkyl;
R² is hydrogen, C₁-C₆-alkyl, C₂-C₆-alkenyl, C₂-C₆-alkynyl, C₃-C₈-cycloalkyl, C₃-C₈-cycloalkyl-C₁-C₆-alkyl, phenyl, phenyl-C₁-C₄-alkyl, phenyl-C₂-C₄-alkenyl or phenyl-C₂-C₄-alkynyl;
wherein the aliphatic moieties of R² are not further substituted or do carry one, two, three or up to the maximum possible number of identical or different groups R^{2a} which independently are selected from:
R^{2a} halogen, OH, CN, nitro, C₁-C₄-alkoxy, C₃-C₈-cycloalkyl, C₃-C₈-halocycloalkyl and C₁-C₄-halogenalkoxy;
wherein the cycloalkyl and/or phenyl moieties of R² are not further substituted or do carry one, two, three, four, five or up to the maximum number of identical or different groups R^{12b} which independently are selected from:
R^{2b} halogen, OH, CN, nitro, C₁-C₄-alkyl, C₁-C₄-alkoxy, C₁-C₄-halogenalkyl, C₃-C₈-cycloalkyl, C₃-C₈-halocycloalkyl and C₁-C₄-halogenalkoxy;
R⁴ is halogen
comprising the following steps:
(i)preparing the compound of formula (I), 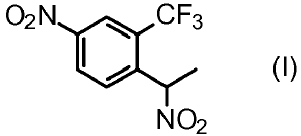 by reacting a compound of formula (II) 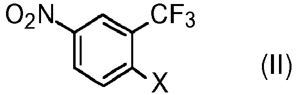 wherein X is a halogen;
with a nitroethane in the presence of a base;
(ii) converting the compound of formula (I) to a compound of formula (III) 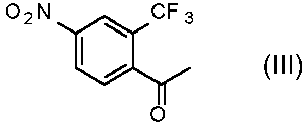 in the presence of an oxidant;
(iii) reacting the compound of formula (III) with a phenol derivative of formula (IV) as described in any one of claims 1 to 9 to give the ketone of formula of formula (V) 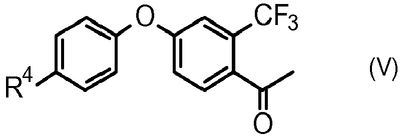
(iv) converting the ketone of the formula (V) to oxiranes (O); 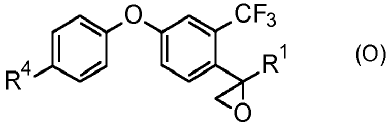 in the presence of trimethylsulfonium methylsulfate of the formula (S2)
(S2) (CH₃)₃S⁺ CH₃SO₄⁻
and a base;
(v) reacting the oxirane (O) with 1H-1,2,4-triazole in the presence of a base to obtain compounds (I), wherein R² is hydrogen (compounds T-1);
and, for obtaining compounds wherein R² is different from hydrogen:
(vi) derivatizing the compound of formula (T-1) of step (v) under basic conditions with R²-LG, wherein LG is a nucleophilically replaceable leaving group; to result in compounds (T-2).
